# EUROPEAN PATENT APPLICATION

(11) **EP 1 180 366 A1**
(43) Date of publication of application: **20.02.2002**
(21) Application number: 00935493.7
(22) Date of filing: 31.05.2000
(51) Int. Cl.: A61K 31/4015, A61K 31/4439, A61P 25/28, A61P 25/16, A61P 43/00, C07D 207/27

(54) **NEURONAL DEATH INHIBITORS**

(30) Priority: 31.05.1999 JP 15172099; 07.06.1999 JP 15933199
(71) Applicant: DAIICHI PHARMACEUTICAL CO., LTD., Chuo-ku, Tokyo 103-8234 (JP)
(72) Inventor: MURASHIMA, Yoshiya, Setagaya-ku, Tokyo 156-0057 (JP); WATABE, Shigeo Daiichi Pharmaceutical Co., Ltd., Tokyo 134-8630 (JP); YOSHII, Mitsunobu, Setagaya-ku, Tokyo 156-0054 (JP)
(74) Representative: Hartz, Nikolai F., Dr.
(86) International application number: JP0003484
(87) International publication number: WO0072844

(57) **Abstract**

The present invention relates to neuronal death inhibitors containing, as its active ingredient, a compound represented by the following formula (1): 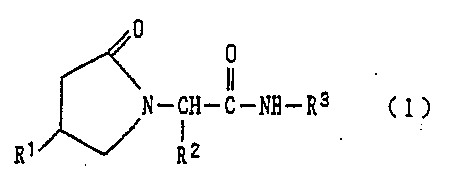 [wherein, R¹ represents a hydrogen atom or a hydroxyl group,
R² represents a hydrogen atom or a methyl group, and
R³ represents a pyridyl group or a phenyl group having 1 to 3 substituents which are the same or different], or acid addition salt thereof.

## Description

### Technical Field

The present invention relates to neuronal death inhibitors, particularly to inhibitors against apoptosisrelated neuronal death and inhibitors against brain neuronal death.

### Background Art

In 1972, Kerr, Wyllie and Currie proposed a concept of apoptosis based on ultrastructural analysis (cell membrane blebbing, nuclear fragmentation and the like), distinguishing physiological cell death from necrosis. Since then, it has been revealed that for eukaryotic cells which repeat cell division, particularly, for those during their growing stage, apoptosis is rather a common pathway in maintaining homeostasis of normal tissues.

Although the same is not equally true of nondividing neuronal cells, it is presumed that an increase in apoptosis of neuronal cells is causative of central neurodegenerative diseases such as Alzheimer's disease, Parkinson's disease and Huntington's chorea and apoptosis takes part in an accelerating phenomenon of neuronal death in cerebral diseases due to ischemia and brain disorders after cerebral hemorrhage. The present inventors therefore recognized the necessity of development of neuronal death inhibitors, particularly inhibitors against apoptosis-related neuronal death and, above all, inhibitors against neuronal death in the brain region.

An object of the present invention is therefore to provide neuronal death inhibitors useful for prevention or treatment of various diseases of the nervous system, particularly inhibitors against apoptosis-related neuronal death, and above all, inhibitors against brain neuronal death.

### Disclosure of the Invention

The present inventors have carried out an extensive investigation on the inhibitory action of various compounds against neuronal death. As a result, it has been found that 2-oxo-1-pyrrolidinylalkylcarboxylic acid amides represented by the below-described formula (1) have excellent inhibitory action against neuronal death, leading to the completion of the present invention.

The present invention therefore provides a neuronal death inhibitor, which comprises, as an active ingredient, a 2-oxo-1-pyrrolidinylalkylcarboxylic acid amide represented by the following formula (1): 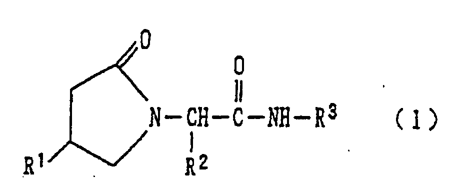 [wherein, R¹ represents a hydrogen atom or a hydroxyl group,
R² represents a hydrogen atom or a methyl group, and
R³ represents a pyridyl group or a phenyl group having 1 to 3 substituents which are the same or different, said substituent(s) on said phenyl group being selected from:
   halogen atoms,
   a trifluoromethyl group,
   a nitro group,
   an acetyl group,
   C₁₋₄ alkyl groups,
   C₁₋₄ alkoxy groups,
   C₁₋₇ alkylthio groups,
   substituted alkylthio groups each represented by the formula: -S-(CH₂)ₙ-CH(R⁴) (R⁵) [wherein, n stands for 1 or 2, R⁴ represents a hydrogen atom or a methyl group, and R⁵ represents a hydroxyl group or an amino group represented by the formula: -N(R⁹) (R¹⁰) (in which, R⁹ represents a hydrogen atom or a methyl group and R¹⁰ represents a methyl, benzyl or substituted benzyl group, or R⁹ and R¹⁰ may be coupled to form a substituted pyrrolidine ring, together with an adjacent nitrogen atom)],
   substituted sulfonyl groups each represented by the formula: -SO₂R⁶ (in which, R⁶ represents an amino group or a C₁₋₃ alkyl group), and
   substituted aminoethoxycarbonyl groups each represented by the formula: -COO(CH₂)ₙ-N(R⁷) (R⁸) (wherein, R⁷ and R⁸ each independently represents a hydrogen atom, a methyl group or an ethyl group)]; or pharmaceutically acceptable acid addition salt thereof.

The present invention also provides the use of the compound of the above-described formula (1) for the preparation of neuronal death inhibitors. The present invention further provides a treating method for inhibiting neuronal death, which comprises administering the compound of the above-described formula (1).

### Brief Description of the Drawings

FIG. 1 illustrates DNA fragmentation in the brains of EL mice one day after administration of nefiracetam; and FIG. 2 illustrates DNA fragmentation in the brains of EL mice five days after administration of nefiracetam.

### Best Mode for Carrying Out the Invention

The compounds of the above-described formula (1) are known compounds described, for example, in Japanese Patent Application Laid-Open (*kokai*) No. Sho 56-2960, Japanese Patent Application Laid-Open (*kokai*) No. Sho 61-280470, Japanese Patent Application Laid-Open (*kokai*) No. Hei 4-160496 and Japanese Patent Publication No. Hei 3-46466. As their action, known are cerebral function improving action (Japanese Patent Publication No. Sho 62-5404), Alzheimer's dementia alleviating action (Japanese Patent Application Laid-Open (*kokai*) No. Hei 5-163144), cerebrovascular dementia alleviating action (Japanese Patent Application Laid-Open (*kokai*) No. Hei 5-163145) and antispasmodic action in EL mice (Nakamoto, et al., "Program and Abstract Reprints of the 17th Annual Meeting of Japanese Society of Neurology (December 7 to 9, 1993, at Nagoya)", p18, No. P1A20), and mitochondrial membrane stabilizing action (Japanese Patent Application No. Hei 8-260649). However, action of these compounds against neuronal death has not yet been revealed at all.

Substituents represented by R³ which exist on the phenyl group in the formula (1) will next be described. The halogen atoms include chlorine and fluorine atoms. The C₁₋₄ alkyl groups include linear or branched alkyl groups such as methyl, ethyl, n-propyl, sec-butyl and n-butyl. The C₁₋₄ alkoxy groups include linear or branched alkoxy groups such as methoxy, ethoxy and isopropoxy. The C₁₋₇ alkylthio groups include linear or branched alkylthio groups such as methylthio, n-propylthio, isopropylthio, sec-butylthio and n-heptylthio.

Examples of the substituted benzyl group represented by R¹⁰ include benzyl groups substituted with 1 to 3 C₁₋₄ alkoxy groups such as dimethoxybenzyl, benzyl groups substituted by 1 to 3 C₁₋₄ alkyl groups such as dimethylbenzyl, and benzyl groups substituted by at least one C₁₋₄ alkyl group and at least one C₁₋₄ alkoxy group, but not greater than 3 in total such as methyl-methoxybenzyl. Examples of the substituted pyrrolidine ring formed by R⁹ and R¹⁰ together with the adjacent nitrogen atom include 2-oxopyrrolidine ring, 3-oxopyrrolidine ring and 2,5-dioxopyrrolidine ring.

Examples of the substituted alkylthio group represented by -S-(CH₂)ₙ-CH(R⁴) (R⁵) include 2-hydroxypropylthio, 2-(N,N-dimethylamino)propylthio, 2-(N-methyl-N-benzylamino)ethylthio, 2-N-methyl-N-(3,4-dimethoxybenzylamino)ethylthio and 2-(2-oxo-1-pyrrolidinyl)ethylthio groups.

The C₁₋₃ alkyl groups represented by R⁶ include linear or branched alkyl groups such as methyl, ethyl and isopropyl. The substituted sulfonyl groups represented by -SO₂R⁶ include aminosulfonyl and methylsulfonyl groups.

The substituted aminoethoxycarbonyl groups each represented by -COO-(CH₂)ₙ-N(R⁷) (R⁸) include 2-(N,N-diethylamino)ethoxycarbonyl group.

Examples of the pyridyl group represented by R³ include 3-pyridyl and 4-pyridyl groups.

Specific examples of the compound preferred as an active ingredient of the medicament of the present invention include:
(1) 2-oxo-1-pyrrolidinylacetic acid 2,6-dimethylanilide,
(2) 4-hydroxy-2-oxo-1-pyrrolidinylacetic acid 2,6-diethylanilide,
(3) 4-hydroxy-2-oxo-1-pyrrolidinylacetic acid 2,6-dimethylanilide,
(4) 2-(2-oxo-pyrrolidinyl)propionic. acid N-3-pyridylamide,
(5) 2-oxo-1-pyrrolidinylacetic acid 4-isopropylthioanilide,
(6) 2-(2-oxo-1-pyrrolidinyl)-1-propionic acid 4-(2-butylthio)anilide,
(7) 2-(2-oxo-1-pyrrolidinyl)propionic acid 4-isopropylanilide,
(8) 2-(2-oxo-1-pyrrolidinyl)propionic acid 2,4-dimethylanilide,
(9) 2-(2-oxo-1-pyrrolidinyl)propionic acid 2,4,6-trimethylanilide,
(10) 2-(2-oxo-1-pyrrolidinyl)propionic acid 2-methoxy-5-methylanilide,
(11) 2-(2-oxo-1-pyrrolidinyl)propionic acid 2,6-dichloroanilide,
(12) 2-pyrrolidone acetamide,
(13) 1-anisoyl-2-pyrrolidinone, and
(14) 4-hydroxy-2-oxo-1-pyrrolidine acetamide.

In addition, compounds disclosed in Table 3 on page 1019 of Japanese Patent Publication No. Hei 3-46466 are preferred.

Out of the above-described compounds, particularly preferred are 2-oxo-1-pyrrolidinylacetic acid 2,6-dimethylanilide [N-(2,6-dimethylphenyl)-2-(2-oxo-1-pyrrolidinyl)-acetamide, general name: nefiracetam], 2-pyrrolidoneacetamide, 1-anisoyl-2-pyrrolidinone, and 4-hydroxy-2-oxo-1-pyrrolidineacetamide.

As the above-described compound (1), any one in the free form or in the form of a pharmaceutically acceptable acid addition salt is usable. The present invention also embraces any hydrate or solvate of the compound. The pharmaceutically acceptable acid addition salts include salts of a mineral acid such as hydrochlorides, hydrobromides, hydroiodides, sulfates, nitrates and phosphates, and organic acid salts such as acetates, maleates, fumarates, citrates, oxalates, succinates, tartrates, malates, mandelates, methanesulfonates, p-toluenesulfonates and 10-camphor-sulfonates.

When the above-described compound (1) has an asymmetric carbon atom, steric configuration of the asymmetric carbon atom is not particularly limited. Any one of a desired optical active substance, a desired mixture of its optical isomers and a racemic mixture may be used. A mixture of desired diastereomers having at least two asymmetric carbon atoms are also usable.

The above-described compounds (1) or acid addition salts thereof are useful as a neuronal death inhibitor, apparently inhibiting DNA fragmentation caused by neuronal death in the mouse brain, which will be described later. Accordingly, the above-described compounds (1) or acid addition salts thereof are useful as a preventive or remedy of diseases associated with an increase in neuronal death, particularly, diseases associated with an increase in apoptosis-related neuronal death, more particularly, diseases associated with an increase in brain neuronal death, for example, central neurodegenerative diseases such as Alzheimer's disease, Parkinson's disease and Huntington's chorea and cerebral diseases due to ischemia and cerebral disturbances after cerebral hemorrhage.

There is no particular limitation imposed on the administration route of the medicament of the present invention and it can be orally or parenterally administered. As the medicament of the present invention, the above-described compound (1) or salt thereof serving as its active ingredient may be administered as is, but it is preferably provided as a pharmaceutical composition containing the compound (1) and pharmaceutically acceptable additives. As the pharmaceutically acceptable additives, usable are excipients, disintegrators, disintegration assistants, binders, lubricants, coating agents, colorants, diluents, bases, solubilizers, solubilizing assistants, isotonizing agents, pH regulators, stabilizers, propellants and pressure-sensitive adhesives. Preparations suited for oral administration include tablets, capsules, powders, fine subtilaes, granules, liquids and syrups. Preparations suited for parenteral administration include injections, drops, suppositories, inhalants and plasters. In the medicament of the present invention, one or more active ingredients can be incorporated.

No particular limitation is imposed on the dose of the medicament of the present invention and it can be selected as needed depending on various conditions such as purpose of the treatment or prevention, type of the disease, age or symptoms of the patient and administration route. Usually, daily dose is about 19 mg to 1000 mg, preferably about 60 to 900 mg, per adult patient. It has been proved that 2-oxo-1-pyrrolidinylacetic acid 2,6-dimethylanilide [general name: nefiracetam] particularly preferred in the present invention is a highly safe compound exhibiting an acute toxicity of 2,005 mg/kg (male mice, oral) (Japanese Patent Application Laid-Open (*kokai*) No. Hei 5-163144).

### Examples

The present invention will hereinafter be described in detail by Examples. It should however be borne in mind that the present invention is not limited to or by them.

### Example 1

Employed were three groups each constituting 6 mice aged 30 weeks, that is, EL mice (EL[s]) given an epileptogenic stimulus once a week and thus having a low threshold, EL mice (EL[ns]) not given an epileptogenic stimulus and free from epileptic seizure at least during observation and DDY mice as a control. Each group was compared with that consisting of 40 to 50 week old mice in order to omit the influence of aging. A section of 10 µm was fixed to a gelatin-coated sliding glass in a cryostat, followed by permeabilization with 0.1% sodium citrate and 0.1% Triton X-100 at 4°C for 5 minutes. DNA fragmentation was detected by polymerizing a fluorescein-labeled nucleotide with the 3'-OH ends of the cleaved DNA by using terminal transferase (TdT). Reaction with an antifluorescein antibody conjugated with peroxidase (PD) or alkali phosphatase (AP) developed color, thereby enabling detection by an optical microscope. As a negative control, TdT(-) and as a positive control, a sample treated with DNAaseI were employed. Nefiracetam was orally administered to the test group (aged 30 weeks, EL[s]) once (10 mg/kg) a day. While thus controlling the occurrence of epileptic seizure, a relationship between the nefiracetam administration terms (1, 3, 5 and 7 days) and appearing frequency of DNA fragmentation was observed.

As a result, DNA fragmentation was detected from neither DDY mice aged 30 weeks (adult) nor those aged 40 weeks (having an influence of aging) which served as a negative control. In the EL mice (EL[s]) having epileptic seizure, whether they were aged 30 weeks or 40 weeks, DNA fragmentation was recognized from their hippocampus and parietal cortex. In the EL mice (EL[ns]) free from epileptic seizure, whether they were aged 30 weeks or 40 weeks, DNA fragmentation was recognized in only a narrower region at a lower frequency than the EL[s]. In their hippocampus, no DNA fragmentation was recognized.

EL mice serve as a model of the region-specific secondary generalized epilepsy. Although having no severe tissue injury, they are known for accelerated production of free radicals in the hippocampus and parietal cortex constituting a partial complex seizure, a rise in NO and site-specific expression of the immediately early genes (*c-fos*, *zif*) during an epileptogenicity acquiring procedure. Localization of DNA fragmentation in the hippocampus and parietal cortex apparently suggests the development of apoptotic procedure of neuronal cells in the hippocampus and parietal cortex of the mice.

DNA fragmentation was recognized in the hippocampus and parietal cortex by single administration of nefiracetam (FIG. 1).

Five days after the administration of nefiracetam, no change was observed in the parietal cortex, but DNA fragmentation disappeared in the hippocampus (FIG. 2). It was therefore revealed that nefiracetam inhibits apoptosis of neuronal cells.

### Industrial Applicability

Compounds (1) or acid addition salts thereof selectively inhibit neuronal death, particularly, apoptosis and are therefore useful as a preventive or remedy of neurodegenerative diseases such as Alzheimer's disease, Parkinson's disease and Huntington's chorea, cerebral diseases associated with ischemia and cerebral disturbances after cerebral hemorrhage.

## Claims

1. A neuronal death inhibitor, which comprises, as an active ingredient, a 2-oxo-1-pyrrolidinylalkylcarboxylic acid amide represented by the following formula (1): 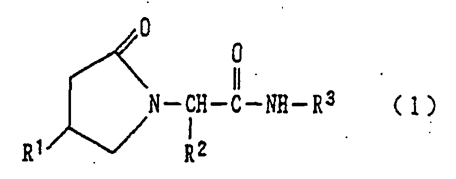 [wherein, R¹ represents a hydrogen atom or a hydroxyl group,
R² represents a hydrogen atom or a methyl group, and
R³ represents a pyridyl group or a phenyl group having 1 to 3 substituents which are the same or different, said substituent(s) on said phenyl group being selected from:
halogen atoms,
a trifluoromethyl group,
a nitro group,
an acetyl group,
C₁₋₄ alkyl groups,
C₁₋₄ alkoxy groups,
C₁₋₇ alkylthio groups,
substituted alkylthio groups each represented by the formula: -S-(CH₂)ₙ-CH(R⁴) (R⁵) [wherein, n stands for 1 or 2, R⁴ represents a hydrogen atom or a methyl group, and R⁵ represents a hydroxyl group or an amino group represented by the formula: -N(R⁹) (R¹⁰) (in which, R⁹ represents a hydrogen atom or a methyl group and R¹⁰ represents a methyl, benzyl or substituted benzyl group, or R⁹ and R¹⁰ may be coupled to form a substituted pyrrolidine ring, together with the adjacent nitrogen atom)],
substituted sulfonyl groups each represented by the formula: -SO₂R⁶ (in which, R⁶ represents an amino group or a C₁₋₃ alkyl group), and
substituted aminoethoxycarbonyl groups each represented by the formula: -COO(CH₂)ₙ-N(R⁷) (R⁸) (wherein, R⁷ and R⁸ each independently represents a hydrogen atom, a methyl group or an ethyl group)]; or pharmaceutically acceptable acid addition salt thereof.

2. A neuronal death inhibitor according to claim 1, wherein the active ingredient is a compound selected from the group consisting of:
(1) 2-oxo-1-pyrrolidinylacetic acid 2,6-dimethylanilide,
(2) 4-hydroxy-2-oxo-1-pyrrolidinylacetic acid 2,6-diethylanilide,
(3) 4-hydroxy-2-oxo-1-pyrrolidinylacetic acid 2,6-dimethylanilide,
(4) 2-(2-oxo-pyrrolidinyl)propionic acid N-3-pyridylamide,
(5) 2-oxo-1-pyrrolidinylacetic acid 4-isopropylthioanilide,
(6) 2-(2-oxo-1-pyrrolidinyl)-1-propionic acid 4-(2-butylthio)anilide,
(7) 2-(2-oxo-1-pyrrolidinyl)propionic acid 4-isopropylanilide,
(8) 2-(2-oxo-1-pyrrolidinyl)propionic acid 2,4-dimethylanilide,
(9) 2-(2-oxo-1-pyrrolidinyl)propionic acid 2,4,6-trimethylanilide,
(10) 2-(2-oxo-1-pyrrolidinyl)propionic acid 2-methoxy-5-methylanilide,
(11) 2-(2-oxo-1-pyrrolidinyl)propionic acid 2,6-dichloroanilide,
(12) 2-pyrrolidone acetamide,
(13) 1-anisoyl-2-pyrrolidinone, and
(14) 4-hydroxy-2-oxo-1-pyrrolidine acetamide.

3. A neuronal death inhibitor according to claim 1, wherein the active ingredient is a compound selected from the group consisting of 2-oxo-1-pyrrolidinylacetic acid 2,6-dimethylanilide, 2-pyrrolidoneacetamide, 1-anisoyl-2-pyrrolidinone, and 4-hydroxy-2-oxo-1-pyrrolidineacetamide.

4. A neuronal death inhibitor according to claim 1, wherein the active ingredient is 2-oxo-1-pyrrolidinylacetic acid 2,6-dimethylanilide.

5. A neuronal death inhibitor according to claim 1, wherein the neuronal death is cell death caused by apoptosis.

6. A neuronal death inhibitor according to claim 1, wherein neuronal death is brain neuronal death.

7. Use, for the preparation of a neuronal death inhibitor, of a 2-oxo-1-pyrrolidinylalkylcarboxylic acid amide represented by the following formula (1): 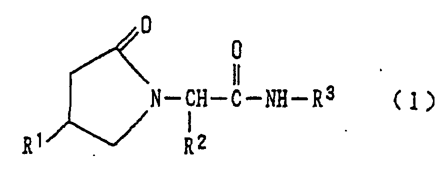 [wherein, R¹ represents a hydrogen atom or a hydroxyl group,
R² represents a hydrogen atom or a methyl group, and
R³ represents a pyridyl group or a phenyl group having 1 to 3 substituents which are the same or different, said substituent(s) on said phenyl group being selected from:
halogen atoms,
a trifluoromethyl group,
a nitro group,
an acetyl group,
C₁₋₄ alkyl groups,
C₁₋₄ alkoxy groups,
C₁₋₇ alkylthio groups,
substituted alkylthio groups each represented by the formula: -S-(CH₂)ₙ-CH(R⁴) (R⁵) [wherein, n stands for 1 or 2, R⁴ represents a hydrogen atom or a methyl group, and R⁵ represents a hydroxyl group or an amino group represented by the formula: -N(R⁹)(R¹⁰) (in which, R⁹ represents a hydrogen atom or a methyl group and R¹⁰ represents a methyl, benzyl or substituted benzyl group, or R⁹ and R¹⁰ may be coupled to form a substituted pyrrolidine ring, together with the adjacent nitrogen atom)],
substituted sulfonyl groups each represented by the formula: -SO₂R⁶ (wherein, R⁶ represents an amino group or a C₁₋₃ alkyl group), and
substituted aminoethoxycarbonyl groups each represented by the formula: -COO(CH₂)ₙ-N(R⁷) (R⁸) (in which, R⁷ and R⁸ may each independently represents a hydrogen atom, a methyl group or an ethyl group)]; or pharmaceutically acceptable acid addition salt thereof.

8. Use according to claim 7, wherein the compound represented by the formula (1) is a compound selected from the group consisting of:
(1) 2-oxo-1-pyrrolidinylacetic acid 2,6-dimethylanilide,
(2) 4-hydroxy-2-oxo-1-pyrrolidinylacetic acid 2,6-diethylanilide,
(3) 4-hydroxy-2-oxo-1-pyrrolidinylacetic acid 2,6-dimethylanilide,
(4) 2-(2-oxo-pyrrolidinyl)propionic acid N-3-pyridylamide,
(5) 2-oxo-1-pyrrolidinylacetic acid 4-isopropylthioanilide,
(6) 2-(2-oxo-1-pyrrolidinyl)-1-propionic acid 4-(2-butylthio)anilide,
(7) 2-(2-oxo-1-pyrrolidinyl)propionic acid 4-isopropylanilide,
(8) 2-(2-oxo-1-pyrrolidinyl)propionic acid 2,4-dimethylanilide,
(9) 2-(2-oxo-1-pyrrolidinyl)propionic acid 2,4,6-trimethylanilide,
(10) 2-(2-oxo-1-pyrrolidinyl)propionic acid 2-methoxy-5-methylanilide,
(11) 2-(2-oxo-1-pyrrolidinyl)propionic acid 2,6-dichloroanilide,
(12) 2-pyrrolidone acetamide,
(13) 1-anisoyl-2-pyrrolidinone, and
(14) 4-hydroxy-2-oxo-1-pyrrolidine acetamide.

9. Use according to claim 7, wherein the compound represented by the formula (1) is a compound selected from the group consisting of 2-oxo-1-pyrrolidinylacetic acid 2,6-dimethylanilide, 2-pyrrolidoneacetamide, 1-anisoyl-2-pyrrolidinone, and 4-hydroxy-2-oxo-1-pyrrolidineacetamide.

10. Use according to claim 7, wherein the compound represented by the formula (1) is 2-oxo-1-pyrrolidinylacetic acid 2,6-dimethylanilide.

11. Use according to claim 7, wherein the neuronal death is cell death caused by apoptosis.

12. Use according to claim 7, wherein neuronal death is brain neuronal death.

13. A treating method for neuronal death inhibition, which comprises administering a 2-oxo-1-pyrrolidinylalkylcarboxylic acid amide represented by the following formula (1): 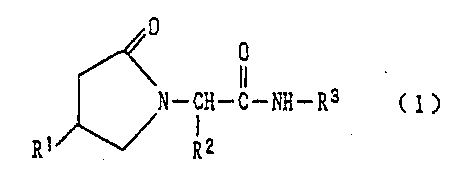 [wherein, R¹ represents a hydrogen atom or a hydroxyl group,
R² represents a hydrogen atom or a methyl group, and
R³ represents a pyridyl group or a phenyl group having 1 to 3 substituents which are the same or different, said substituent(s) on said phenyl group being selected from:
halogen atoms,
a trifluoromethyl group,
a nitro group,
an acetyl group,
C₁₋₄ alkyl groups,
C₁₋₄ alkoxy groups,
C₁₋₇ alkylthio groups,
substituted alkylthio groups each represented by the formula: -S-(CH₂)ₙ-CH(R⁴) (R⁵) [wherein, n stands for 1 or 2, R⁴ represents a hydrogen atom or a methyl group, and R⁵ represents a hydroxyl group or an amino group represented by the formula: -N(R⁹)(R¹⁰) (in which, R⁹ represents a hydrogen atom or a methyl group and R¹⁰ represents a methyl, benzyl or substituted benzyl group, or R⁹ and R¹⁰ may be coupled to form a substituted pyrrolidine ring, together with the adjacent nitrogen atom),
substituted sulfonyl groups each represented by the formula: -SO₂R⁶ (wherein, R⁶ represents an amino group or a C₁₋₃ alkyl group), and
substituted aminoethoxycarbonyl groups each represented by the formula: -COO(CH₂)ₙ-N(R⁷) (R⁸) (wherein, R⁷ and R⁸ each independently represents a hydrogen atom, a methyl group or an ethyl group)]; or pharmaceutically acceptable acid addition salt thereof.

14. A method according to claim 13, wherein the compound represented by the formula (1) is a compound selected from the group consisting of:
(1) 2-oxo-1-pyrrolidinylacetic acid 2,6-dimethylanilide,
(2) 4-hydroxy-2-oxo-1-pyrrolidinylacetic acid 2,6-diethylanilide,
(3) 4-hydroxy-2-oxo-1-pyrrolidinylacetic acid 2,6-dimethylanilide,
(4) 2-(2-oxo-pyrcolidinyl)propionic acid N-3-pyridylamide,
(5) 2-oxo-1-pyrrolidinylacetic acid 4-isopropylthioanilide,
(6) 2-(2-oxo-1-pyrrolidinyl)-1-propionic acid 4-(2-butylthio)anilide,
(7) 2-(2-oxo-1-pyrrolidinyl)propionic acid 4-isopropylanilide,
(8) 2-(2-oxo-1-pyrrolidinyl)propionic acid 2,4-dimethylanilide,
(9) 2-(2-oxo-1-pyrrolidinyl)propionic acid 2,4,6-trimethylanilide,
(10) 2-(2-oxo-1-pyrrolidinyl)propionic acid 2-methoxy-5-methylanilide,
(11) 2-(2-oxo-1-pyrrolidinyl)propionic acid 2,6-dichloroanilide,
(12) 2-pyrrolidone acetamide,
(13) 1-anisoyl-2-pyrrolidinone, and
(14) 4-hydroxy-2-oxo-1-pyrrolidine acetamide.

15. A method according to claim 13, wherein the compound represented by the formula (1) is a compound selected from the group consisting of 2-oxo-1-pyrrolidinylacetic acid 2,6-dimethylanilide, 2-pyrrolidoneacetamide, 1-anisoyl-2-pyrrolidinone, and 4-hydroxy-2-oxo-1-pyrrolidineacetamide.

16. A method according to claim 13, wherein the compound represented by the formula (1) is 2-oxo-1-pyrrolidinylacetic acid 2,6-dimethylanilide.

17. A method according to claim 13, wherein the neuronal death is cell death caused by apoptosis.

18. A method according to claim 13, wherein neuronal death is brain neuronal death.
